# EUROPEAN PATENT APPLICATION

(11) **EP 1 340 521 A2**
(43) Date of publication of application: **03.09.2003**
(21) Application number: 03250772.5
(22) Date of filing: 06.02.2003
(51) Int. Cl.: A61N 5/06

(54) **Medical light therapy apparatus**

(30) Priority: 27.02.2002 GB 0204525
(71) Applicant: Whale, Jon, Seaton, Devon EX12 2YB (GB)
(72) Inventor: Whale, Jon, Seaton, Devon EX12 2YB (GB)
(74) Representative: Brown, Michael Stanley

(57) **Abstract**

Medical therapy apparatus comprises one or more transducers (3) for emitting radiation by means of dielectric resonance when subjected to an electrical stimulus, generating means (9) for providing the stimulus and control means (8, 10) for controlling the generating means (9) whereby radiation emitted from the transducers (3) is controlled so as to synchronise with the radiation generated in a selected diseased or injured area of a body and thereby stimulate or attenuate emissions of radiation by the body in the diseased or injured area.

## Description

### Field of the Invention

This invention relates to medical therapy apparatus.

### Background to the Invention

For thousand of years, balms and tablets containing green beryllium aluminium silicate (emerald gem stone powder) and dark blue aluminium oxide (blue sapphire powder) have been and are indeed today being prescribed by physicians in some countries for pain, contusions, infections, dermatological conditions, digestion and gastric disease, etc. These mineral medicines, like sand, do not dissolve and cannot be absorbed into the body's biochemistry. They, therefore, function on unconventional justifications compared to biochemical medications.

The present invention relates to low voltage medical apparatus that does not come into direct contact with the patient's body. The invention utilises the principle of dielectric resonance which is the subatomic transfer of energy between discrete body mass. This principle is used to manipulate and/or modulate cellular biological energy via a sub-molecular level of valence electron modulation. Modulating electrons in living cells will adjust, increase or decrease the biological energy or life force of the cells.

A very large percentage of presented injuries and diseases support excessive biological energy. For example, contusions, fractures, burns, carcinomas, lymphomas, melanomas, prostate cancer, dermatological diseases, rheumatoid arthritis, diabetes mellitus and associated pathology, liver disease and many other common conditions, including bacterial infections, can be accompanied by localised vasodilation, hyperthermia, hyperperfusion, hypermetabolism and or hypervascularisation: all high energy conditions that are often accompanied with discomfort and pain.

It is an object of the present invention to provide an improved form of medical therapy apparatus that can be used for the treatment of many different conditions, such as those outlined above.

### Summary of the Invention

According to the present invention there is provided therapy apparatus comprising one or more transducers for emitting radiation by means of dielectric resonance when subjected to an electrical stimulus, generating means for providing the stimulus and control means for controlling the generating means whereby radiation emitted from the transducers is controlled so as to synchronise with the radiation generated in a selected diseased or injured area of a body and thereby stimulate or attenuate emissions of radiation by the body in the diseased or injured area.

The apparatus of the present invention preferably comprises low voltage medical lamps containing crystalline mineral substrates that are frequency-modulated and these lamps are dielectric resonance transducers used to target and treat diseased or injured parts of the body by inducing calibrated quantitative and qualitative energy.

The apparatus of the present invention provides excellent efficient management and palliative care procedures for a wide range of symptom, injuries, neurological or physical disease and iatrogenic disease by artificially manipulating and modulating the biological energy of the diseased parts without producing any side effects. Furthermore, by targeting the relevant glands and organs, the patient's haematology and biochemistry can be adjusted and improved. The patient's experience is always pleasant regardless of the symptoms or disease being treated. As they receive treatment, over 75% of patients report relief from their pain, irritations, uncomfortable or distressing feeling, emotions and symptoms. The rapid beneficial effect is scientifically supported, for example, by monitoring with radiometric imaging or pathological laboratory analysis as well as by other diagnostic methods, observations and subjective reports.

The frequency of the energy induced is dictated by the colour of the mineral substrate fitted in the transducer. For example, ruby has a resonant frequency wavelength λ of around 625 ηm (nanometres) (the frequency of the colour red) and the energy emitted is subjectively hot, whereas emerald has a green frequency wavelength λ of around 565 ηm and its energy is subjectively cold. The mineral substrate contained in a special chamber inside the transducers (lamps) is electronically excited to resonate with low voltage electronic pulses.

The dielectric resonance frequency of emerald substrates is in the green spectrum with a wavelength λ of around 565 ηm and blue sapphire has a wavelength λ of around 400 ηm. Dielectric resonance transducers containing green beryllium aluminium silicate and dark blue aluminium oxide crystalline substrates are extremely effective for reducing localised hyperthermia, hyperperfusion, hypermetabolism and vasodilation as well as dramatically attenuating pain. This type of transducer is electronically modulated at slow analgesic brainwave frequency of sleep (1.5 Hz). They are employed for the management of perhaps 90% of all presented diseases or injuries.

The inverse complementary management equivalent to the above procedure is dielectric resonance transducers containing red aluminium chromium oxide (ruby) having a wavelength λ of around 625 ηm, or orange silicon dioxide (carnelian with a wavelength λ of around 610 ηm) together with carbon (diamond with a wavelength λ of around 470 ηm), i.e. crystalline substrates electronically modulated at a faster frequency (16.5 Hz). This combination can rapidly and effectively reduce hypothermia, vasoconstriction, hypometabolism, increasing local temperature, circulation and biological energy. It is used for management of thrombosis, venus ulcers, phlebitis, chilblains, varicose veins, oedema and related pathology.

The substitution of ruby with carnelian crystalline substrates produces a softer treatment for conditions midway between the two procedures cited above: examples are asthma, cerebral thrombosis, embolism or haemorrhage, paralysis, dementia, polymyalgia, vitiligo, muscular/ligamentous strain, adhesive capsulitis, disc prolapse, osteoarthritis, rheumatic disease, allergies and many others.

Transducers filled with emerald and blue sapphires fitted with a green filter at a frequency of 1.5 Hz and are effective for reducing localised hyperthermia, hyperperfusion, hypermetabolism and vasodilation as well as dramatically attenuating pain. They are used to cool and pacify nerves, tissue, muscles, organs or endocrine glands. They can be used for burns, new injuries, inflammation and soreness, dermatitis, eczema, digestion and gastric condition, i.e. conditions where cellular activity is too hot and over-active.

Transducers filled with blue sapphires fitted with a dark violet filter at a frequency of 3.3 Hz are a powerful sedative, analgesic, anti-spasmodic, (muscle and nerve relaxant). They are used to calm, relax and pacify nerves, tissue, muscles, organs or endocrine glands and for pain associated with nerve damage and irritation, sciatica, arthritis, psoriasis, post injury and surgical trauma, anxiety, shock, stress, phobias, migraine, drug and alcohol addiction, emotional trauma, emotional and physical exhaustion. These are all conditions where there is too much nervous activity, tension, anxiety, distress or irritation. The use of blue sapphire transducers reduces heart rate and blood pressure and therefore they are nearly always used for anxious and nervous patients.

Transducers filled with carnelians and diamonds fitted with an orange filter at a frequency of 8.5 Hz effectively reduce vasoconstriction, hypometabolism, and increase circulation and biological energy. They act as an anti-coagulant. They are used to induce stimulating warm biological energy, activity and circulation in tissue, muscles, organs or endocrine glands. They are thus used for chronic fatigue, depression, asthma, eczema, oedema, varicose ulcers, circulation and various blood conditions, numbness and muscular atrophy, cartilage and disc problems. They are also used to stimulate under active organs and glands and are often used with elderly patients, as they increase biological energy.

Transducers filled with rubies and diamonds fitted with a red filter at a frequency of 16.5 Hz rapidly reduce hypothermia, vasoconstriction, hypometabolism, increasing local temperature, circulation and biological energy. They are used to induce stimulating hot biological energy, activity and increase circulation in tissue, muscles, organs or endocrine glands. They are used with caution for anaemia, amenorrhea, constipation, chilblains, low blood pressure, rheumatism, heart disease, varicose ulcers, and varicose veins.

The above examples are those most commonly used in clinical practice. For ease of understanding, the subjective effects experienced by patients as a result of the use of various crystalline substrates are as follows:-
a) Ruby - heating, drying, energizing, expanding,
b) Carnelian - cooling, moist, harmonizing, anti-allergenic,
c) Citrine - warm, enlivening, cleansing,
d) Emerald - cold, unifying and solidifying, analgesic, relaxant, anti-spasmodic,
e) Topaz - cool, satisfying, antiseptic,
f) Diamond - stimulating, invigorating, clarifying, anti-depressant,
g) Sapphire - cool, tranquillising, soothing, analgesic, sedative, anti-spasmodic,
h) Chrysoberyl - soft, deep penetrating heat, drying, energizing,
i) Zircon - coldest of all, used in cases of excessive heat, and
j) Amethyst - hypnotic, narcotic, tranquillising, soothing, analgesic, sedative, anti-spasmodic.

Many combinations or mixtures of the various crystalline substrates can be used so as to combine their individual medically beneficial properties. For example, the heat-inducing properties of ruby can be combined with the analgesic properties of blue sapphire and this combination of crystalline substrates will be used to reduce vasoconstriction and related pain or discomfort.

The present invention substantially improves the medical effects of traditional crystalline mineral medical preparations. The treatment apparatus provides medical benefits whereby the energy emitted from precious gem stones and synthetic mineral substrates is greatly increased and easily controlled so as to provide precision application for the management of a wide range of medical symptoms, diseases, and injuries, as well as for neurological diseases and iatrogenic disease. Furthermore, the invention permits precision targeting of the diseased or injured parts of the patient.

### Brief Description of the Drawings

Figure 1 is a diagrammatic representation of one form of apparatus in accordance with the present invention, the apparatus including a control instrument,
Figure 2 is a block schematic diagram of the control instrument of the apparatus shown in Figure 1 and the associated power supply,
Figure 3 is an exploded view of a transducer (lamp) forming part of the apparatus shown in Figure 1,
Figures 4 and 5 illustrate embodiments of the apparatus in accordance with the present invention for individual treatments,
Figure 6 illustrate further embodiments of the apparatus in accordance with the present invention for special purpose medical apparatus,
Figure 7 show further embodiments of the apparatus in accordance with the present invention for special purpose medical apparatus, and
Figure 8 shows a number of further embodiments of the apparatus in accordance with the present invention for special purpose medical apparatus for the preparation and manufacture of electronically energised medicines, elixirs, balms and emollients for the treatment of diseases, symptoms and injuries.

### Description of the Preferred Embodiments

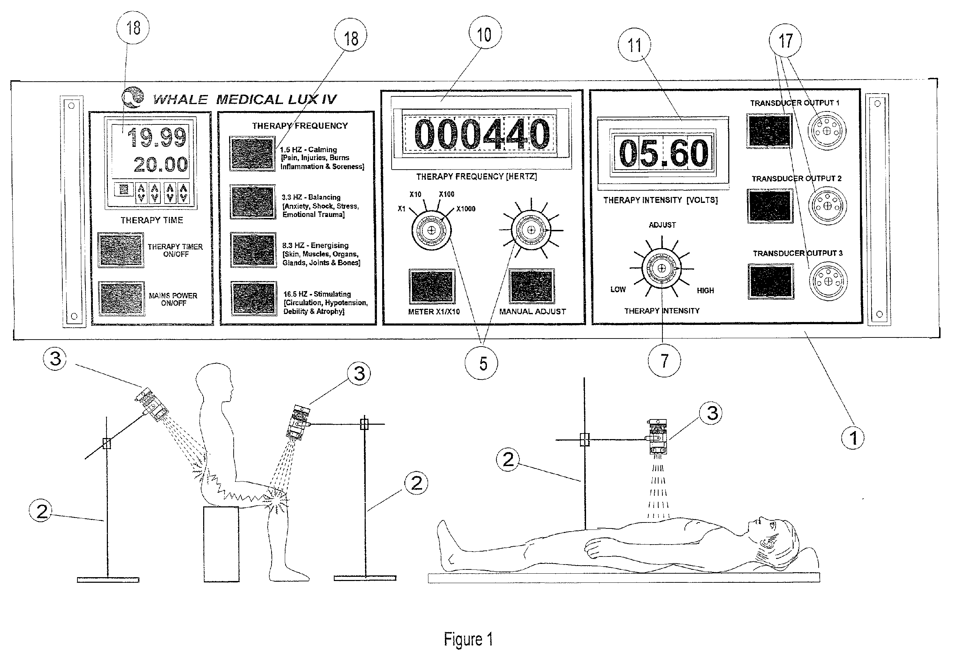

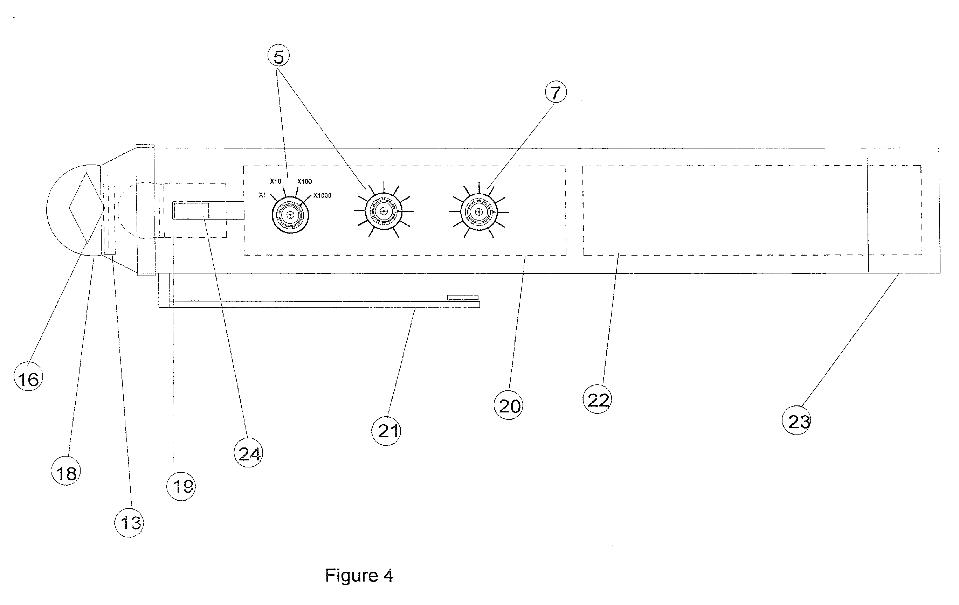

Referring now to Figures 1 and 4 of the drawings, there is shown an apparatus for carrying out treatment on the human body. The apparatus includes a control instrument and power supply 1 and adjustable support stands 2. Transducers (lamps) 3 are mounted on the support stands 2 which can be so positioned as to enable the transducers 3 to target any area of the patient's body. The control instrument 1 has a display panel on which there are a number of operating elements that can be adjusted to provide the frequency and treatment intensity suitable to treat the patient's presented disease, injury, symptoms or complaint.

The methods of carrying out treatments for the numerous diseases, injuries, symptoms or complaints are listed below.

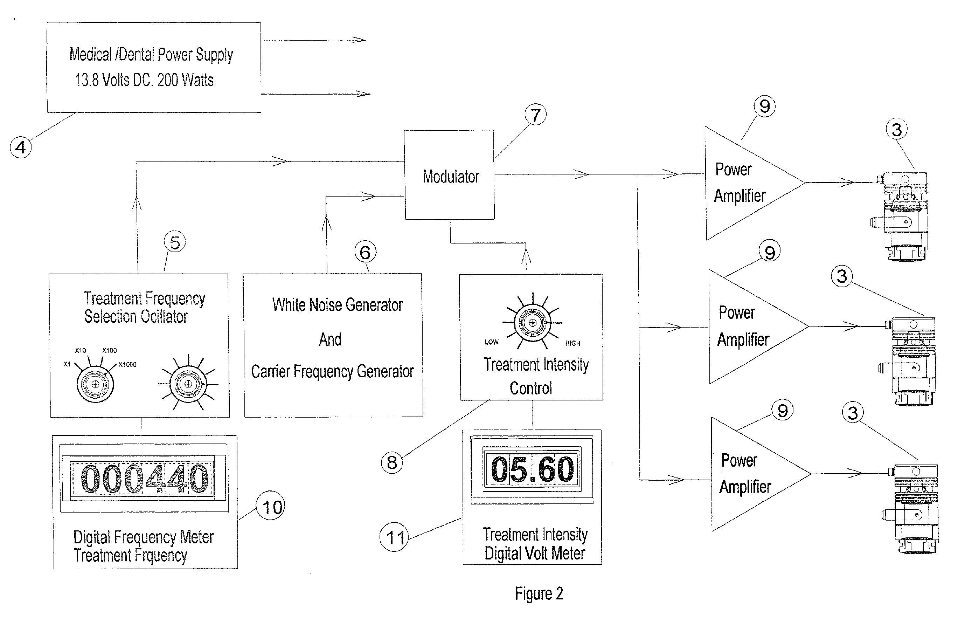

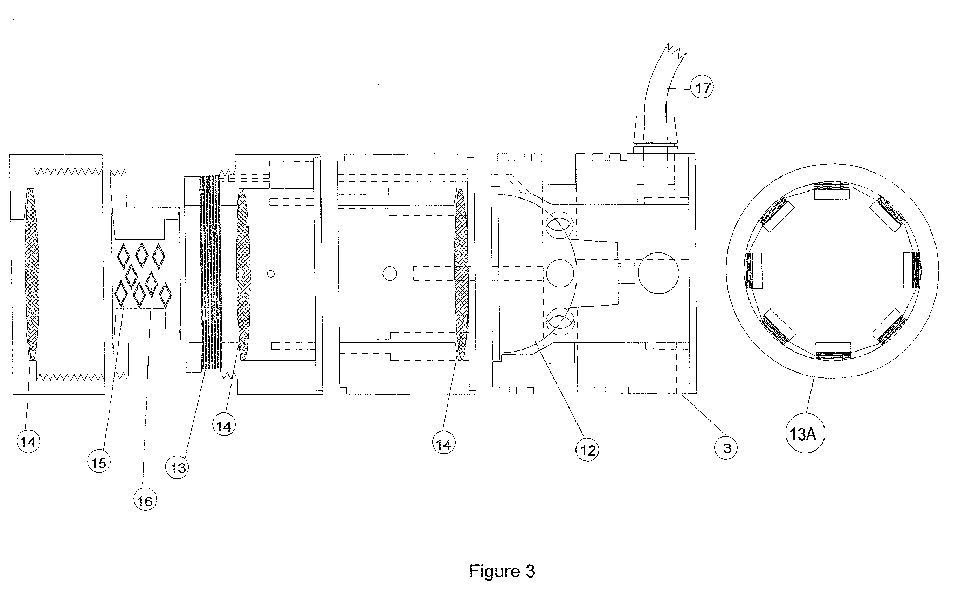

The 13.8 volts D.C. power supply 4, shown in Figure 2, is certified for medical and dental use. The power supply 4 provides 13.8 volts power to illuminate a light bulb 12 (Figure 3) inside a transducer (lamp) 3. As shown in Figure 2, the 13.8 volts D.C. power supply 4 also supplies the power for a treatment frequency oscillator 5, a white noise generator 6, a modulator 7, a treatment intensity control 8, transducer amplifiers 9, a digital frequency meter 10, and a treatment intensity meter 11. The output from the treatment frequency oscillator 5 provides the means to drive the modulator 7, which is used to modulate the white noise signal from the noise generator 6. The output level of the modulator 7 is controlled by the treatment intensity control 8, which is monitored and displayed by the digital treatment intensity meter 11.

The variable frequency and amplitude-modulated white noise output of the modulator 7 is feed directly into power amplifiers 9, which are used to drive the induction coils 13, inside the transducers (lamps) 3. The modulated white noise power signal delivered by the power amplifiers 9 to the induction coils 13 inside the transducers (lamps) 3 can be controlled, both in frequency and amplitude, by the treatment frequency oscillator 5 and the treatment intensity 8 control knobs. These controls, together with the calibrated digital treatment frequency meter 10 and the precision treatment intensity meter 11, permit accurate scientific calibrated treatment monitoring of the treatment given to the patient.

The mechanical design and construction of the transducers (lamps) 3 is such that the infra red and ultra violet rays that may be generated by the light bulb are filtered out and the patient is not exposed to the infra red and ultra violet rays. This filtering is achieved by the use of soda lime glass filter lenses 14 and dichroic lamp bulbs 12. The mechanical construction of the transducers (lamps) 3 is such that the electromagnetic waves and the heat generated by the transducers (lamps) 3 are contained and dissipated inside the metal housings of the transducers (lamps) 3. The patient and the practitioner are thus not exposed to any electromagnetic radiation, infra red or ultra violet rays.

The light beam is polarized and its frequency (colour) is controlled by placing natural crystalline substrates in a containment chamber 15. inside the transducers (lamps) 3 as follows:
Ruby = Red @ 625 nm.
Carnelian = Orange @ 610 nm.
Citrine = yellow @ 590 nm.
Emerald = Green @ 565 nm.
Yellow/Blue Sapphire = Blue @ 485 nm.
Diamond = Indigo @ 470 nm.
Dark Blue Sapphire = Violet @ 400 nm.

These crystallised mineral substrates give precision calibrated control of the frequency (colour) of the light beam. In addition to this the crystallised mineral substrates inside the transducers are electromagnetically stimulated with electronic pulses and modulated frequencies by the induction coil 13 that is arranged around the chamber 15. The frequencies used are duplicates of the natural frequencies produced in a healthy human body.

The apparatus includes a copper coil 13 wound around the mineral chamber 15. Other multiple inductors can be arranged around the mineral chamber 15 in a manner similar to the construction of an electric motor armature cage 13A. Such an arrangement provides more inductive power with less stray electromagnetic radiation and results in faster treatment times and requires a smaller quantity or size of mineral substrates to reproduce the same medical effects. This arrangement would be suitable for natural minerals and also for some types of synthetic crystalline mineral substrates. Synthetic substrates can be machined into circular discs or lenses.

In Figure 4 of the drawings there is shown a form of apparatus in a low power miniature construction for personal use suitable to be carried in the pocket or handbag. The apparatus housing 23 contains a battery power pack 22, a miniature electronic printed circuit board 20, a light source 19, an induction coil 13 or 13A, an optional removable glass retainment dome 18, and mounted crystalline substrates or gem stones 16. The electronics can be a miniaturised version with similar operation functions as already described with reference to Figure 2 above. An on/off switch 24 controls the light source and the electronic circuitry. The output frequency is adjusted by controls 5 and the intensity by controls 7.

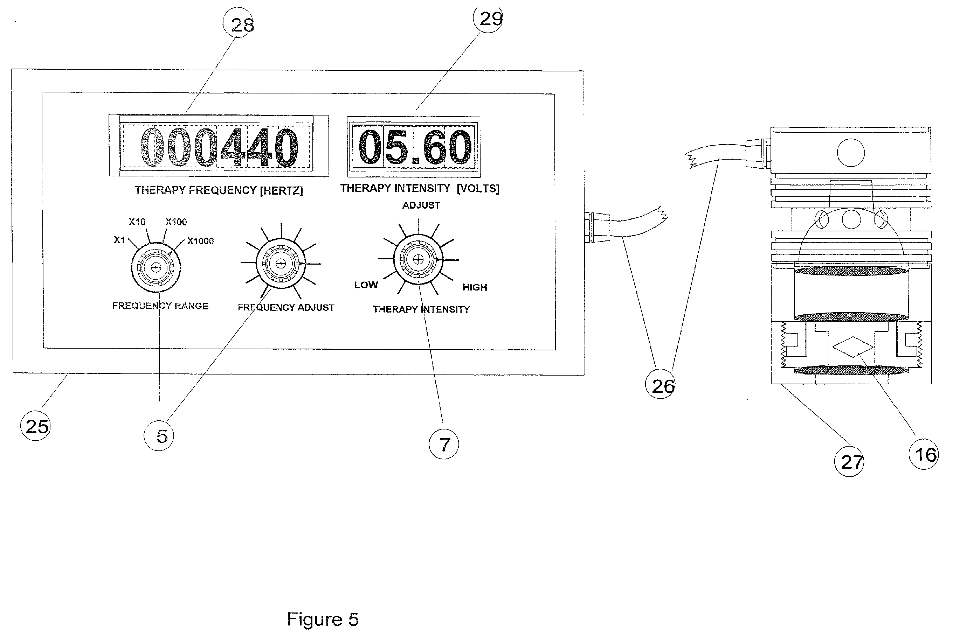

Figure 5 of the drawings shows an apparatus of medium power small construction for personal use suitable for patients to treat themselves for management of chronic conditions when not attending a clinic or hospital. An enclosure 25 contains an electronic circuit similar to that already described in relation to Figure 2. A small lightweight lower power transducer 27 is connected to the apparatus via a cable 26. A treatment digital frequency meter 28 and a treatment intensity meter 29 along with the means for adjusting the treatment frequency and the treatment intensity work on the same principles as already described in connection with the embodiment shown in Figure 2. The device shown in Figure 5 is suitable for patients' personal use. It is economical to manufacture, being small and portable. The same principles, circuitry and design parameters are employed.

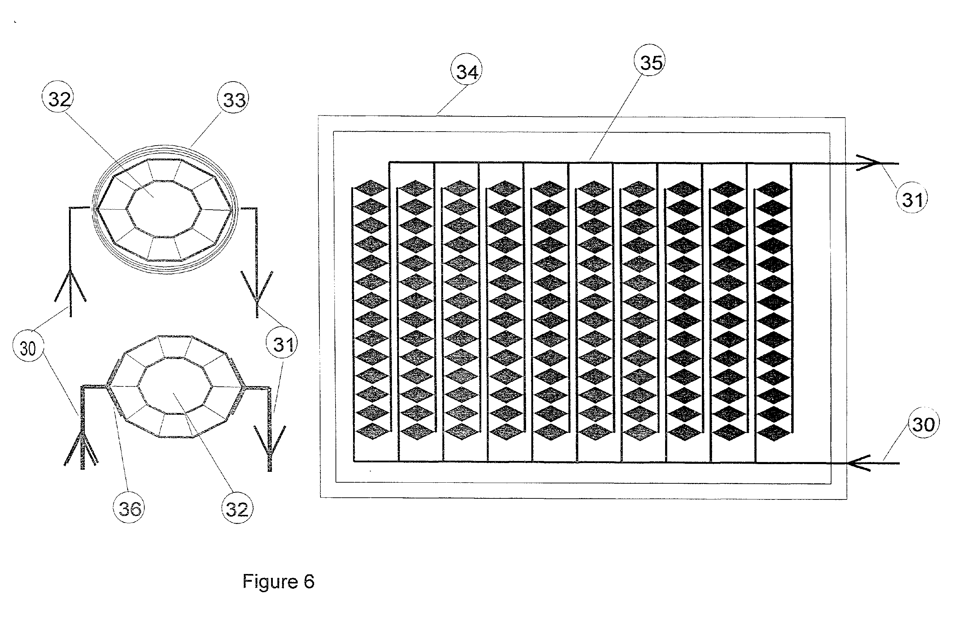

Figure 6 of the drawings shows more details of the apparatus by way of example only. The techniques for exciting the gem stones or crystalline substrates 32 with induction from a copper wire coil 33 can be substituted by directly applying the output signal voltages and frequencies of Figure 2 directly to the gem stones or crystalline substrates 32 when they are mounted between suitable electrode contacts 36. This method is extremely useful when applied to embodiments of the present invention for the smaller more portable personal therapy apparatus illustrated in Figures 4 and 5. It is also most suitable for larger transducers designed and constructed for specialised medical purposes, for example, the treatment of patients suffering with burns that cover large parts of the body. This would enable a matrix of gem substrates 35 to be mounted over a large area in an enclosure containing a light source 34. This arrangement is suitable for both natural minerals and also for some types of synthetic crystalline substrates.

The anodes 30 and the cathodes 31 of Figure 6 are the means by which the treatment frequencies and voltages are applied to cause the gem stones or crystalline substrates to resonate and emit by way of dielectric resonance their beneficial medical properties. Figure 6 also illustrates another method for exiting crystalline substrates or gem stones to emit more energy. These methods can substitute the transducers outlined in Figure 3.

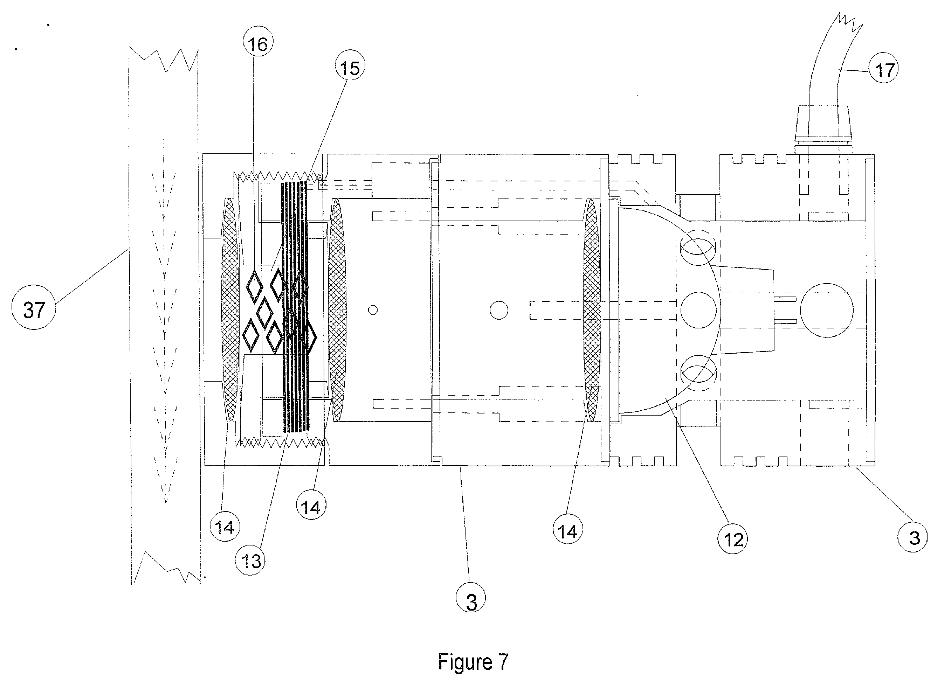

Figure 7 of the drawings show an embodiment of the invention in the form of an apparatus by which blood transfusions, plasma transfusions, intravenous medications and dialysis treatment materials can be treated with dielectric resonance energy. The blood, plasma, intravenous medication or other intravenous modalities of treatment are passed through a tube 37 that is made of glass or some other suitable material. The tube 37 is mounted or positioned in closed proximity to the output of a suitably constructed transducer for this purpose. As the blood, plasma or other intravenous liquid medication or essential nutrients pass though the tube 37, they will become energised by way of dielectric resonance thus imparting the beneficial energy of gem stones or crystalline substrates directly to the patient. Thus, Figure 7 illustrates the means by which blood transfusions, plasma transfusion, intravenous medications and dialysis treatment can be treated with dielectric resonance.

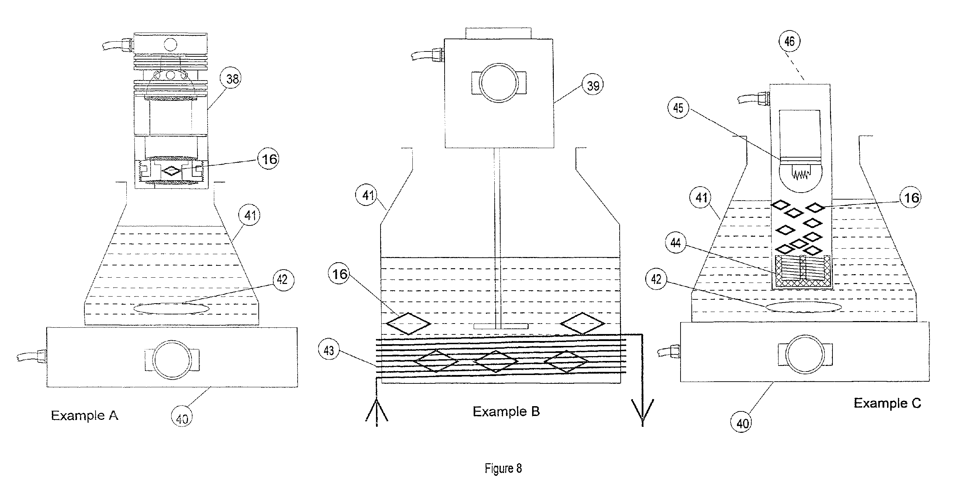

Figure 8 of the drawings shows three forms of apparatus in accordance with the present invention for means by which special purpose medical apparatus is arranged for the preparation and manufacture of electronically energised or charged medicines, elixirs, balms and emollients by the scientific principles of dielectric resonance. The principles of dielectric resonance, when appropriately applied directly to diseased or injured parts of a human body, have profound and rapid beneficial healing properties. The same principles can be applied via other substrates such as medicines, elixirs, balms and emollients. The storage life of such preparations is limited, as the energised substrates will slowly dissipate their induced and accumulated energy into the local environment by dielectric resonance.

One example of a method of preparation is the energisation of an aqueous cream used to placate and protect skin disease and injuries such as burns, psoriasis, eczema and dermatitis. All of these conditions respond exceptionally well to recovery when treated with transducers using emerald and blue sapphire crystalline substrates. They also respond to an aqueous cream that has been energised with transducers containing emerald and blue sapphire crystalline substrates. Figure 8 illustrates three methods of preparing medications.

In example A of Figure 8, a vessel 41 containing the medical substrate is being agitated by a magnetic stirrer 40 and 42. The agitated medical substrate is energised by a transducer 38 containing the prescribed gem stones or crystalline substrates. The time required to fully energise the medical substrate depends on the volume, specific gravity and the molecular weight of the vessel's contents and also the power of the transducer that is being employed.

In example B of Figure 8, a vessel 41 containing the medical substrate together with the prescribed gem stones or crystalline substrates 16 is being agitated by an electric motor stirrer 39. An induction coil 43 is arranged around or underneath the vessel together with or without a light source. The agitated medical substrate is energised by electronic frequencies and voltages applied to the induction coil.

In example C of Figure 8, a vessel 41 containing the medical substrate is being agitated by a magnetic stirrer 40 and 42. The agitated medical substrate is energised by a transducer 46 containing the prescribed gem stones or crystalline substrates 16, a light source 45 and an induction coil 44.

The active transducers and instruments generally do not come into any physical contact with patients and the transducers (lamps) operate with only a 12 volts D.C. 20 watt light bulb inside a housing machined from solid metal. 12 volt, direct current transducers (lamps) are used so that the patient is not exposed to any 50 Hertz mains frequency electromagnetic emissions, which is the case with most traditional colour therapy or infra red or ultra violet therapy lamps. Each transducer produces an insignificant emission at 5 centimetres distance of only 4 to 8 thousandth of a gauss which is a hundred times less than that produced by a typical ladies electric shaver.

The apparatus in terms of power consumption, electromagnetic emissions and applications is several hundred times improved upon and safer compared to other similar medical and therapeutic lamps that are used both in hospitals and beauty salons etc. For example:
1] Common colour therapy lamps which are normally 240 volts A.C. - 50 Hz and generally around 200 watts.
2] Infra red heat therapy lamps are 240 volts A.C. - 50 Hz and generally around 2000 - 3000 watts.
3] Ultra violet tanning and medical photo therapy lamps are 240 volts A.C. - 50 Hz and generally around 2000 - 4000 watts.

These traditional devices have very high levels of 50 Hz electromagnetic emissions of up to several hundred gauss.

Contact with the patient's body or removal of clothes or dressings is often unnecessary, but it can be helpful, and at least, when possible, the area of skin should be exposed for treatment. With infected or open wounds, a light protective dressing can be left in place to maintain sterility. Dielectric resonance energy can pass through clothing and dressings, but the colour-filtered light cannot. Applying the transducers close to the unclothed skin will permit shorter treatment times and greater accuracy at targeting internal organs or glands. Treatment with the transducers improves a patient's complexion, energy levels and mood, and these changes should be apparent after twenty minutes of treatment. In many years of research and with thousands of patient's receiving treatment, not a single incident of side effects or complaint has been reported.

Although transducers are completely effective on their own, they can, however, be valuably employed in conjunction with surgery, allopathy, psychiatry, physiotherapy, osteopathy, chiropractic and the various complementary support therapies. The following examples demonstrate the simplicity and effectiveness of treatment procedures for various conditions.

Acne. There are various types of acne, therefore the treatments vary according to which type is being treated. Sometimes the acne compared to unaffected skin can be very hot as measured with an infra red scanner. This is particularly the situation where infection is present. When this is the case, treatment should be given using emerald and sapphire with a green filter. The effected area should be treated from a distance of approximately 5 to 10 cm. and the frequency to use is 1.5 Hz. In other cases, the condition can be cleared by stimulating the local circulation using diamond and carnelian with an orange filter at 8.5 Hz. It is also recommended that the patient's spleen is treated with diamond and carnelian with an orange filter at 8.5 Hz. This will improve the biological activity of all of the blood components, increase the activity of the immune defence system and increase the efficiency of the spleen and the cardiovascular system. Also, in some cases, a cooling calming treatment to the liver with emerald and sapphire at 1.5 Hz is very helpful. This will improve the efficiency of the liver and reduce the amount of unwanted toxins and proteins in the blood.

Allergies. Generally speaking, most allergies are manageable. The treatment to apply will depend largely on the symptoms. Diseases such as asthma, eczema, acne, hay-fever, IBS, colitis and so on are caused or thought to be caused by sensitivity to environmental conditions, certain types of foods or chemical contaminants. To treat allergies, refer to this index under the appropriate subject that matches the patient's complaints. Most allergies can be treated and cleared up by using transducers containing diamond and carnelian with an orange filter at 8.5 Hz. Treatment should be given to the spleen.

Alopecia. This is best treated using two transducers containing dark blue sapphires and a dark violet filter and frequency of 1.5 Hz. The patient's head should be targeted from a distance of approximately 10 to 20 cm. away from the head, or closer if only a small area is affected. The treatment should be repeated on a weekly or fortnightly basis until re-growth of the hair becomes apparent. With patients that have been treated by this method, hair growth has become apparent after two treatments. This treatment will induce a state of profound relaxation and the patient should be cautioned beforehand. The patient should also be advised to take things easy for the next few days following treatment and instructed to enjoy the post-treatment relaxation period. Should this condition fail to respond to treatment using sapphire to the effected area, then treatment with diamond and carnelian with an orange filter should be tried. This will have the effect of stimulating the local circulation and increasing local biological energy. As this is a stimulating treatment it should be performed before midday, as it may interfere with the patient's sleep.

Alzheimer's Disease. Senile dementia and its related problems can be greatly assisted and patients respond rapidly, with improvements to memory, humour, interests and activities. Use one or two transducers containing a mixture of diamonds and carnelian with an orange filter to target the patient's spleen with a frequency of 8.50 Hz. This will effectively stimulate and tone up the patient's blood as it passes through the spleen on the way to liver. The circulation of the blood will carry and distribute the energy to all the organs and glands and extremities of the patient's body including the brain. The patient should be made comfortable, sitting or lying on an examination couch. The transducers should be arranged to target the spleen from a distance of approximately 1 - 5 cm. The duration of the treatment should be for approximately 20 minutes once a week or every second week. Maintenance treatment can be given one a month.

Anorexia. This is easily treated. When a person is under weight, emerald has the ability to increase the weight of the patient and slow down the metabolic rate. Another effect of emerald is that it is employed for diarrhoea. However, with anorexia patients, this is not a problem and this effect of emerald is beneficial. Furthermore, emerald is used for digestive problems, irritable bowel syndrome, gastric ulcers, stomach ulcers, indigestion, and many other symptoms relating to the assimilation of food. Many anorexia patients are prone to vomiting after eating food. Therefore, the stomach should be targeted with transducers containing emeralds and dark blue sapphires with a green filter at a frequency of 1.5 Hz and treatment voltage of 12 volts. This will cool down and completely relax the stomach area. Examination of the liver, spleen, stomach, intestines and thyroid glands either by hand or by infra red scanning should be done to ascertain whether these are over or under active (too hot or cold). In the case where the liver is very hot, then it should also be treated with emeralds and dark blue sapphires in order to reduce its activity.

Anxiety. This and panic are very easily treated using transducers containing dark blue sapphires with a violet filter. The frequency to use is 3.3 Hz and the patient should be made comfortable lying down on an examination table. The transducers should be arranged to target each side of the patient's head from a distance of approximately 15 to 20 cm. The treatment voltage to be set is 12 volts or less. With very sensitive patients, the treatment voltage should be reduced to six volts or less. This treatment will induce a state of profound relaxation and the patient should be cautioned beforehand. The patient should also be advised to take things easy for the next few days following treatment and enjoy the post-treatment relaxation period.

Considerations should also be given to the patient's liver, since nervous patients may have an over-active liver with too much adrenaline and bile production. Great benefit therefore can be gained by cooling down the liver with a transducer containing emeralds and blue sapphires with a green filter. The frequency should be at 1.5 Hz. If a third transducer is used to treat the liver, then the same frequency of 3.3 Hz should be used to treat the patient's head using the third output from the instrument. If the patient is overweight and the liver is cold and congested, then treatment should be given to the spleen using diamonds and carnelian at the same frequency. The treatment voltage should be 12 volts. This is a profoundly relaxing and enjoyable treatment.

Contra-indications: patients who have experienced a heart attack can, when subjected to this profound relaxation treatment, associate the induced relaxation subconsciously with the distress caused by their heart attack. If this occurs, the patient's heart rate will increase. Patients suffering with heart conditions should be treated with transducers containing diamonds and carnelian and an orange filter to their spleen. Very industrious, extrovert, active energetic patients that never bother to relax, do not enjoy being relaxed and may complain about this treatment even though it is extremely beneficial for them.

Arthritis. There are several different types of arthritis. Rheumatoid arthritis, osteo-arthritis, sero-negative arthritis, infective arthritis and ankylosing spondylitis respond well. In the first place the pain should be relieved with emeralds and sapphires using a green filter at the frequency of 1.5 Hz, the treatment voltage should be set to 12 volts and treatment distance should be 5 to 10 cm. away from the affected areas. All associated muscles should be relaxed with the same treatment to remove the stress to the affected joints. Considerations should be given to the liver, spleen, kidneys and blood pressure. If the liver is over-active or inflamed then it should be calmed down with emeralds and sapphires, using a green filter at the treatment voltage of 12 volts. There is also benefit in treating the spleen and energising the blood. If this is done in the early stages of the disease then it is possible to slow down or reverse the development of these diseases.

Asthma. This is easily treated. With children and young adults, the practitioner can expect close to 100% positive response. With older people, the success rate will also be very good and the practitioner can expect around 60 to 80% without recourse to other medications. However, older chronic asthma sufferers require more treatment sessions. The management procedure for asthma is to use diamonds and carnelian with an orange filter fitted in the transducers. The treatment voltage should be set for 12 volts and duration of the treatment should be for approximately 20 minutes. With youngsters of the age group up to six, this treatment should be given with caution and duration should be around 5 to 10 minutes. This is a stimulating treatment, so young children can possibly become hyper active. Hyperactivity can be treated by calming down the liver with emeralds and dark blue sapphires. In acute cases, the treatment should be given to the lungs at the front of the chest and at the patient's back.

Atrophy. This can occur with diabetes, strokes, cardiovascular problems and old age. Transducers containing diamond and carnelian and an orange filter at 8.5 Hz should be used when tissue, organs and glands are atrophied. When extremities of the body are involved, such as the lower legs and feet, then ruby and diamond should be employed at a frequency of 16.5 Hz with a red filter.

Bacterium infections. Infected cuts, injuries, bones, organs and glands should be treated with transducers containing emerald with a green filter at 1.5 Hz. With bacterial infections, the site of the infection will almost always be at a higher temperature and emerald will rapidly reduce the temperature and the biological activity and energy of the invading organism. This will permit the white blood cells to overrun and kill off the infection. This treatment is not much use in the case of viral infections, apart from relieving soreness. The following is not necessary if the patient's general health is good, but it is very helpful in the case of older patients. Stimulate the dermis circulation local to the infection and also stimulate the spleen with diamond and carnelian with an orange filter at 8.5 Hz. This is very important if the infection relates to bones or organs such as the kidneys, liver, brain and so on. It may also be helpful to stimulate the thymus gland as this will increase lymphocyte production - if the count is low.

Back Pain. Even with severe disc prolapse conditions, remarkable results can be expected with only one or two treatments. As inflammation of the spinal nerve causes irregularities in the muscle tension of the back, legs and abdomen, which in turn causes distortion of the skeletal frame, before commencing any treatment for lower back pain, the patient should be laid out flat on an examination table with the ankles close together to check pelvis alignment and the skeletal alignment. For example, with sciatica, one leg will appear to be longer than the other, often with a mismatch of several centimetres. This can easily be checked by measuring the displacement at the ankle joints (media malleolus). After treatment this should be repeated to observe that there has been an improvement in pelvis alignment and the muscle tension.

The problem with irregular muscle tension is that it distorts the spinal column which in turn applies more pressure to the injured area of the spinal nerve causing even more injury and inflammation, which in turn causes more muscle tension. This sets up a kind of positive feedback system which will push and pull the discs and joints out of alignment, causing the patient deep distress. Using the apparatus in accordance with the present invention to cool and calm down inflamed nerve sections is the fastest and safest way to intercept and destroy this feedback loop of increasing muscle tension.

As with all kinds of pain, the first treatment that should be considered is to use transducers containing dark blue sapphires and a violet filter. Where nerve inflammation is involved, such as with sciatica or slipped disc problems, then emeralds should also be included in the transducer and the filter should be changed to green. The treatment duration is 20 minutes and treatment voltage should be set to 12 volts. This treatment will cool down and reduce the nerve inflammation as well as relaxing the related muscles. Relaxing the muscles takes the pressure off the disc and it can and does often realign itself naturally.

Inflammation of the spinal nerve causes local heat and aggressive biological activity, this can easily be detected with an infra-red scanner and it will measure several degrees Celsius hotter than unaffected parts of the spine, furthermore a sensitive practitioner should be able to detect the heat or excess biological activity of the inflamed section by examination using the fingers or the palm of the hand. This is an important point as pressure and inflammation to spinal nerve will cause pain in other areas of the body such knees and hips. For example, it is not very helpful to treat the pain that may occur in one leg of the patient suffering from sciatica without treating the inflamed section of spinal nerve.

Typically, with severe sciatica, the patient can expect immediate relief from pain and mobility difficulties with only one treatment. Treatment of the back can be carried out by sitting the patient sideways on a chair or stool. It is very practical to ascertain the mobility and pain levels of the patient prior to commencing treatment. Typically, the practitioner will get them to bend forwards, backwards and sideways whilst standing. He or she will also get them to lift each leg off the floor to establish their movement and pain thresholds and ask them to give a number value from 0 to 10 for their pain level. Once their limits have been established, the practitioner will commence treatment and, during the course of the treatment and at the end, he or she will get them to stand up and repeat the exercise to ascertain the effectiveness of the treatment given. The practitioner will ask the patient each time for a pain level value, and the number they quote should decrease as their treatment progresses. Patients treated in this way should be able to leave the surgery pain free and with greatly improved mobility.

Patients suffering with a disc prolapse can, once the pain and inflammation has been attended to, be treated with diamonds and carnelian with an orange filter. This will stimulate local circulation and increase the biological energy around the disc prolapse. The affected disc and associated joints should be targeted from a distance of 5 cm. and a frequency of 8.5 Hz. The treatment level should be 12 volts for a duration of 20 minutes. At the same time a second and third transducer containing emeralds and dark blue sapphires should be positioned either side of the disc prolapse under treatment with the transducer containing diamonds and carnelian (using the second and third outputs of the instrument). These two transducers are used to relax the muscles and back so that the joints can expand and allow the disc to naturally realign and relocate itself.

Another common cause of back pain is abdominal surgery such as hernia or hysterectomy. Post-surgery trauma can cause abdominal muscles to pull up tight with tension, which in turn will be reflected in counter-balancing tension of the muscles of the patient's back causing severe pain and mobility difficulties. Therefore, it is essential to ascertain whether or not the patient has received abdominal surgery and whether the area of the surgery is tender, sensitive or painful. If this is the case then it is essential to treat surgical lesion or scar tissue with emeralds and dark blue sapphires with a green filter.

Note: When back pain fails to respond to emerald and/or sapphire, it will most often respond well to a mixture of 50% ruby and dark blue sapphire using a red filter at 1.5 Hz.

Bedsores. Pressure wounds or bedsores are best treated with transducers containing diamond and carnelian with an orange filter at 8.5 Hz. This will improve local circulation and raise the biological activity of the affected dermis. A more practical solution, where possible, is to treat the bed-confined patient with the appropriate treatment for the conditions that are incapacitating them in the first place. If the sores are painful then it may be necessary to use emerald and sapphire with a green filter in the first place.

Biliousness. The practitioner will target the liver, stomach, duodenum, gallbladder and intestines with transducers containing emerald and sapphire and a green filter at 1.5 Hz. This treatment may tend to cause slight constipation.

Blindness. Loss of sight due to thrombosis or vasoconstriction etc. in the optic nerve is treated with transducers containing diamond and carnelian with an orange filter at 8.5 Hz. The practitioner will target the optic nerve area of the effected eye from the side of the head around the temple area. This condition is associated with diabetes and treatment to the spleen should be given to improve the quality and biological energy of the blood components and of the cardiovascular system as well as all of the organs and glands.

Burns and scalds respond rapidly using a mixture of emeralds and sapphires, with a green filter fitted to the transducer. The frequency to use is 1.5 Hz, and the treatment voltage should be set to 12 volts. The duration of the treatment is 20 minutes and can be repeated as required. The affected tissue should be targeted from distance of between 5 and 15 cm. This treatment will rapidly cool down tissue and reduce pain. It will also accelerate and improve the healing process. Another beneficial factor of this treatment is that it will reduce the formation of scar tissue, reduce the risk of infection and clear up infection, if present. With old burns or scar tissue, using diamonds and carnelian with an orange filter at 8.5 Hz will improve the appearance.

Candida. The practitioner will use transducers containing diamond and carnelian with an orange filter at 8.5 Hz to target the spleen. This will improve the biological energy of the blood and the immune defence mechanisms. Patients with candida will be very low in energy and may also have ME, depression or chronic fatigue syndrome. Their assemblage point will almost certainly be found in a low location in the liver. This should be corrected. The treatment to the spleen will have the effect of shifting the assemblage point up from the liver by 4 to 8 centimetres.

Capsulitis. The practitioner will use transducers containing diamond and carnelian with an orange filter at 8.5 Hz to target the effected area. He or she will also relieve the pain and relax the associated muscles groups with blue sapphire and a dark violet filter, and use emerald where inflammation is present.

Carpal Tunnel Syndrome. The practitioner will use transducers containing emerald and sapphire with a green filter at 1.5 Hz. This will often solve the problem often with a single treatment.

Cartilage Problems. Transducers containing diamond and carnelian with an orange filter at 8.5 Hz will be used to target the affected area.

Catarrh. Transducers containing diamond and carnelian with an orange filter at 8.5 Hz will be used to target the lungs and spleen.

Chilblains. The practitioner will target the affected area with transducers containing ruby and diamond with a red filter to stimulate circulation and increase cellular biological energy. Ears should be treated from behind, so that the light beam shines only on the ears, not on the head. This treatment will normally clear up chilblains with one session.

Cirrhosis of the Liver. Benefit can be gained by cooling down the liver with a transducer containing emeralds and blue sapphires with a green filter the frequency should be at 1.5 HZ.

Colds. Colds take hold when the patient's biological energy is low and cellular membrane resilience is low. It is effective to use a transducer containing diamond and carnelian with an orange filter at 8.5 Hz to target the spleen. Using a transducer containing emeralds and blue sapphires with a green filter, the frequency should be at 1.5 Hz to relieve sore throats, nose and ears.

Colitis. The practitioner will use two transducers containing emeralds and blue sapphires with a green filter and the frequency should be at 1.5 Hz close to the skin. The practitioner will target the areas of the patient's discomfort plus the liver and gallbladder.

Coma. Transducers containing diamond and carnelian are used with an orange filter at 8.5 Hz to target the spleen. This will increase the biological energy and efficiency of the spleen, blood, liver, heart, lungs, brain and kidneys.

Conjunctivitis. Transducers containing diamond and carnelian with an orange filter will be used at 8.5 Hz to target the eyes.

Cystitis. A transducer containing emeralds and blue sapphires will be used with a green filter at 1.5 Hz directly on to the affected area. This treatment will normally clear up cystitis with one or two sessions.

Depression. Depression is most often due to a low entry angle of the assemblage point. This can easily be corrected with a transducer containing diamond and carnelian with an orange filter at 8.5 Hz to target the spleen. See, however, anxiety and stress, which can be confused with depression. Clinical depression is due to the patient's assemblage point dropping into the liver area. The frontal brain energy levels will be low. The assemblage point must be lifted up. This can be achieved by energising the blood via the spleen with transducers containing diamond and carnelian with an orange filter at 8.5 Hz to target the spleen. Also, the frontal brain can be energised using a transducer containing diamonds.

The following treatment is not usually used these days, but is mentioned as a comparison and a safe alternative to electro-convulsive shock therapy. The practitioner will use only small diamonds and target the top part of the patient's forehead from a distance of 30 cm. Only 3 to 6 volts of treatment intensity will be used with an indigo filter.

Dermatitis. This is often caused by chemical irritation and the source of the contamination should be eliminated. If the dermis is hot and irritated, use transducers containing emerald and sapphire with a green filter at 1.5 Hz. Otherwise, stimulate the local circulation and increase the biological activity of the dermis with transducers containing diamond and carnelian with an orange filter at 8.5 Hz.

Earache. Use one or two transducers containing emerald and sapphire with a dark violet filter at 1.5 Hz. Target the side of the head and ears from a distance of 16 centimetres. Advise the patient that, as a result of the treatment, he or she will feel relaxed and calm for a day or so. If infection is present, include yellow sapphire.

Eczema. This most often responds if the circulation and biological energy is increased. Use a transducer containing diamond and carnelian with an orange filter at 8.5 Hz to target the area affected and also the spleen. If the affected area is irritated, hot and sore, then a transducer containing emerald and sapphire with a green filter at 1.5 Hz should be used. Eczema can be due to internal organ imbalances and malfunctions, or it can be due to external irritation and sensitivity. If the eczema is considered to be due to internal problems, then check the patient's liver and spleen energy levels, and consider treating them. If the liver is hot, then use emerald and sapphire at 1.5 Hz to calm it down. Eczema is often accompanied by asthma and the treatment then should be the same as for asthma.

Eye Injuries/Infections. For injuries and bacterium infections, use transducers containing emerald and sapphire and a green filter at 1.5 Hz. For viral infections, use a transducer containing diamond and carnelian with an orange filter at 8.5 Hz.

Gastrointestinal Problems. Use two transducers containing emerald and sapphire with a green filter at 1.5 Hz close to the skin. Target the liver, gall bladder, stomach, duodenum, or intestines, etc. This is a cooling, calming treatment.

Gastric and Duodenum Ulcers. Use two transducers containing emerald and sapphire with a green filter at 1.5 Hz close to the skin. Target the liver, gall bladder, stomach, duodenum, or intestines, etc.

Hay Fever. Sensitivity increases as the patient's biological energy decreases. Diamonds and carnelian increase the cellular energy. The practitioner will use transducers containing diamond and carnelian with an orange filter at 8.5 Hz to target the lungs from the patient's back and chest front. He will also consider treating the spleen with diamond and carnelian to improve the blood quality. For the irritation, a transducer containing emerald and sapphire and a green filter can be used at 1.5 Hz to treat the nose and throat.

Heat Rash. The practitioner will use transducers containing emeralds and blue sapphires with a green filter and the frequency should be at 1.5 Hz close to the skin. This is a cooling, calming treatment.

Herpes. The practitioner will use diamond and carnelian on the spleen at 8.5 Hz and apply citrine to the mixture and to any part of the body affected by the herpes virus. He will use emeralds and blue sapphire for acute genital herpes.

Hepatitis. Two transducers containing emerald and sapphire will be used with a green filter at 1.5 Hz close to the skin. The practitioner will target the liver, gall bladder, stomach, duodenum, or intestines, etc. This is again a cooling, calming treatment. The spleen will be treated with transducers containing diamond and carnelian with an orange filter at 8.5 Hz.

Hyper Activity. Two transducers containing emerald and sapphire will be used with a green filter at 1.5 Hz close to the skin. The practitioner will target the liver, gall bladder, stomach and duodenum. This is again a cooling, calming treatment.

Inflammations. Two transducers will be used close to the injury and will contain emeralds and blue sapphires with a green filter. The frequency should be at 1.5 Hz. This is again a cooling, calming treatment and is not suitable for oedema.

Influenza. At the first sign of influenza, or for protection against influenza, the practitioner will energise the blood via the spleen using transducers containing diamond and carnelian with an orange filter at 8.5 Hz. For treating the throat, nose and chest, transducers containing emeralds and blue sapphires will be used with a green filter, and the frequency should be at 1.5 Hz.

Itching. One or two transducers containing emeralds and blue sapphires will be used with a green filter and the frequency should be at 1.5 Hz. Target the affected area from a distance of 16 centimetres. Advise the patient that, as a result of the treatment, they will feel relaxed and calm for a day or so.

Injuries. All injuries and contusions will benefit from treatment using one or two transducers containing emeralds and blue sapphires with a green filter; the frequency should be at 1.5 Hz. The affected area is targeted from a distance of between 5 and 10 centimetres.

Insomnia. Two transducers containing emeralds and blue sapphires are used with a green filter and the frequency should be at 1.5 Hz. An over-active liver will cause insomnia and, therefore, the liver is targeted.

I.B.S. Two transducers containing emerald and sapphire are used with a green filter at 1.5 Hz close to the skin. The liver, gall bladder, stomach, duodenum, or intestines, etc. are targeted. This is a cooling, calming treatment. The areas of the patient's discomfort are targeted.

Jaundice. Two transducers containing emerald and sapphire are used with a green filter at 1.5 Hz close to the skin. The liver, gall bladder, stomach, duodenum, or intestines are targeted. This is a cooling, calming treatment for the liver. The spleen can also be treated with transducers containing diamond and carnelian with an orange filter at 8.5 Hz.

Ligament Injuries. All types of ligament injuries, sport-related or otherwise, are easily and rapidly addressed. The procedure involves relaxing all associated muscles and energising the actual damaged area to promote a rapid healing. Transducers containing emerald and blue sapphire are used to relax all of the muscles that relate to the site of the injury with a frequency of 1.5 Hz and a green filter. If necessary, two transducers can be used at the same time. For example, if the injury is around the knee, one transducer is used on each side of the knee joint to relax the upper and lower leg muscles.

Once the muscles are relaxed and soft to the touch, the practitioner can employ a third transducer containing carnelian and diamonds with an orange filter at the same frequency and will use it to target the injured, torn or sprained area. The third transducer is applied for 20 minutes, and then switched off. The practitioner will not switch off the transducers containing emerald and sapphire but will leave these operating for a further 5 to 10 minutes.

M.E. This condition is precipitated and is sustained due to the patient's assemblage point dropping into the liver area or lower. The psychological factors are not important to induce normal health. When the patient's Assemblage Point descends down into the liver area, the liver will become seriously disturbed and will not function correctly. The patient will feel tired, lacking in energy and the body will not respond to his or her mental commands. A transducer containing diamond and carnelian will be used at a frequency of 8.3 Hz and the patient's spleen will be targeted. The treatment duration should be for a minimum period of 20 minutes. The method of locating the Assemblage Point and the significance thereof is explained in "The Catalyst of Power - The Assemblage Point of Man" published by Findhorn Press, ISBN 1-899171-73-8.

Migraine. Two transducers filled with sapphire will be used at 1.5 Hz with a blue filter. The practitioner will target each side of the head from a distance of 16 centimetres and will advise the patient that, as a result of the treatment, he or she will feel relaxed and calm for a day or so. Migraine can also be due to an energy blockage in the throat, as well as stomach, liver and spleen imbalances. Assemblage Point misalignment will also be an indicator and a contributing factor. The practitioner will enquire of the patient's medical history and other symptoms to establish the root cause of the pain and will treat accordingly. If treating the throat area, a transducer containing blue topaz will be used with a blue filter at 8.3 Hz for 20 minutes.

Neuralgia. One or two transducers containing blue sapphire will be used with a dark violet filter at 1.5 Hz for 20 minutes. The practitioner will target the site of the pain from a distance of 5 to 10 centimetres and will advise the patient that as a result of the treatment, he or she will feel relaxed and calm for a day or so.

Neuritis. Transducers containing emerald and sapphire will be used with a green filter at 1.5 Hz for 20 minutes.

Oedema. The practitioner will treat the legs and arms to improve circulation and increase biological energy. The soles of the feet and palms of the hands can also be targeted. This will cause pleasant tingling sensations. Chronic oedema can by improved by energising the spleen, for this the practitioner will use a transducer containing diamond and carnelian and an orange filter at 8.3 Hz for 20 minutes.

Osteomyelitis. Bacterial infections of bones and the related pain can be treated. Infection of the jaw bone due to dental root canal fillings is quite common and can cause a variety of symptoms elsewhere in the body. Bones can also become infected as a result of surgery, accidents and other infections which spread to the bone and marrow. Acute bone infection should always be referred to a doctor for antibiotic treatment. Bone infection and the toxins produced by the bacteria can make the patient feel very sick. Antibiotics are not very effective at treating bone infections as the blood supply is limited by the porosity of the bone structure and this prevents the delivery of the antibiotics to the infected site. This then can lead to chronic osteomyelitis.

Chronic bone infection is best treated, assuming that the infected area is hot and inflamed (bacterium activity), with a cooling treatment using transducers containing emerald and sapphire to reduce the swelling, reduce the pain and arrest the activity and subdivision (growth) of the bacteria present. A green filter will be used and a frequency of 1.5 Hz. The affected area will be targeted as closely as possible.

The patient's blood, immune defence system and cardiovascular system should be energised to assist in the destruction of the bacterium. For this, the practitioner will use one or two transducers containing a mixture of diamonds and carnelian with an orange filter to target the patient's spleen with a frequency of 8.50 Hz. This will effectively stimulate and tone up the patient's blood as it passes through the spleen on the way to liver. The circulation of the blood will carry and distribute the energy to all the organs and glands and extremities of the patient's body including the brain. The transducers should be arranged to target the spleen from a distance of approximately 1 to 5 cm. The duration of the treatment should be for approximately 20 minutes once a week or every second week. Treatment to the thymus gland should also be considered to increase the lymphocyte activity and count of the blood.

Pain. All pain should be treated and can be dramatically reduced with transducers containing emerald and sapphire and a green filter at 1.5 Hz. Pain is the first consideration, living with constant pain places great energy demands on the patient. Low energy is a feedback loop that increases pain. A pain loop can be broken by intercepting it at any of the feeding nerve pathways. The practitioner will advise the patient that as a result of the treatment, he or she will feel relaxed and calm for a day or so. Chronic pain lowers the location of the assemblage point (and can raise it to an anxiety position). This should, therefore, be corrected.

Pleurisy. One or two transducers containing diamond and carnelian will be used with an orange filter at 8.5 Hz at close proximity to the effected area of the lungs. This condition will often clear with a single treatment. Consideration should be given to treating the spleen.

P.M.T. Many patients have been successfully treated for this common condition and they have reported that their condition did not reoccur. It is necessary to get the patients to attend the clinic at the time that they are experiencing the symptoms. Two transducers containing only blue sapphire will be used with a dark violet filter. The practitioner will make the patient comfortable on a treatment couch and, with the abdomen area exposed, will target the area with the transducers from a distance of about 15 to 20 centimetres for a duration of about 15 to 20 minutes at a frequency of 3.3 Hz.

Golden topaz, emerald, sapphire, moonstone (or carnelian) and a small amount of diamond can be used as a mixture to treat a variety of symptoms relating to the womb, including menopausal problems and fibroids. The practitioner will target the womb for 20 minutes at 3.3 Hz using a blue filter. To slow excessive bleeding, emerald should first be used at 1.5 Hz with a green filter for 20 minutes. Fibroids sometimes respond very positively to green chrysoberyl at 1.5 Hz using a green filter for 20 minutes.

Polymyalgia rheumatica. This condition can be swiftly treated with transducers containing diamond and carnelian fitted with an orange filter. The frequency should be set to 8.5 Hz. The patient's spleen should be targeted with two transducers. The soles of the feet and the palms of the hands should also be treated.

Psoriasis. With skin problems one can expect and get at least an 80% positive response. In the case of youngsters with skin problems (and asthma), one can expect close to 100% positive response, often with a single treatment.

Example A. Where the patient's skin cells are behaving erratically with continual flaking skin, the condition can be rapidly calmed down and eliminated by calming down the nervous electrical activity in the dermis and the brain. This is achieved by using a transducer containing blue sapphire with a dark violet filter, at a frequency of 1.5 Hz, to target the crown of the patient's head. Sapphire rays eliminate pain and have a profound calming and soothing effect on the patient. This is an extremely pleasant experience. It calms down the biological energy of the patient and skin cells. If the patient's skin is extensively affected, two transducers placed so that the light shines on each side of the patient's head will induce profound relaxation. As the treatment proceeds, the patient's blood pressure and heart rate become calmer, and skin irritation subsides. In most cases, this treatment is sufficient to cause the condition to clear up. If the patient's skin is only affected in a small area, then the transducers are generally only used to target the area of skin requiring treatment.

B. The above treatment will not be effective where the patient's skin has a wart like or lumpy appearance. This condition requires and responds rapidly to a stimulating transducer treatment using a yellow filter and citrine gem stones simulated at a faster frequency of 8.5 cycles per second. This treatment is also effective for warts and a single 10 minute exposure will get rid of warts.

C. Neither of the above treatments will work in the case where the patient's liver is hot, sweaty and overactive and the skin condition looks both like psoriasis and eczema, perhaps with fluid discharging from the dermis. These types of symptoms respond rapidly to a calm cooling transducer treatment targeting the patient's liver, using a transducer containing emeralds and sapphire with a green filter at a frequency of 1.5 Hz. This treatment cools and calms down the overactive biological energy of the patient's liver in minutes, and this can easily be confirmed by physically examining the liver with the hand. In addition, the patient will report that they feel better as the treatment progresses, together with a remarkable improvement in their complexion. Also, the patient's blood pressure and heart rate become calmer.

D. Certain types of acne, eczema, dermatitis, and urticaria will respond to the above treatments and others will not, and will require a stimulating treatment to improve the condition of the blood and circulation. Depending on the patient's overall symptoms and examination results, the following treatment may be given to the affected dermis and/or the liver, and/or the spleen. The spleen is responsible for the quality control of the blood and in many diseases, it has been found that targeting the patient's spleen with a stimulating treatment using an orange transducer containing a mixture of white diamonds and orange carnelian using a stimulating frequency will clear up many skin and other common conditions. This treatment increases the vitality, biological energy and efficiency of the spleen and all of the blood that passes through it as the treatment proceeds. The energised blood passes or imparts its energised condition to all of the patient's organs, dermis and bones. For those patients who are rundown and low in biological energy, this treatment induces a profound feeling of well-being. This particular treatment is not only effective for skin problems, it also is effective for oedema (often accompanied with skin problems), varicose ulcers, asthma, sinus problems, senile dementia, strokes and many other diseases, including some diabetic conditions.

Prolapse Disc. The following treatment is extremely effective for all types of disc problems. Two transducers containing blue sapphire are used at 1.5 Hz. The transducers are placed on either side of the affected joints. This will relax the tense muscles associated with the pain and take the pressure off the joint. One or two treatments will be given on a weekly basis and the practitioner will then introduce a third transducer containing diamond and carnelian with an orange filter at the same frequency. This third transducer will be used to target the cartilage of the joint under treatment. This will stimulate local circulation and increase the resilience and biological energy of the cartilage.

Repetitive Strain Injury. The following treatment is extremely expeditious for all types of RSI. Two transducers containing blue sapphire are used at 1.5 Hz. The transducers are placed on either side of the affected joints. This will relax the tense muscles associated with the pain and take the pressure off the joint. The practitioner will give one or two treatments on a weekly basis and then introduce a third transducer containing diamond and carnelian with an orange filter at the same frequency. This third transducer will be used to target the cartilage of the joint under treatment. This will stimulate local circulation and increase the resilience and biological energy of the cartilage.

Rheumatism. At first, the practitioner will try a transducer containing ruby and diamond using a red filter at 8.3 Hz for a few minutes. If there is no reduction in pain, a transducer containing emerald and sapphire will then be used with a blue or green filter for 20 minutes at 8.3 Hz.

Scleroderma. The treatment for this condition is to stimulate the local circulation using a transducer containing diamond and carnelian with an orange filter at a frequency of 8.5 Hz. The patient's spleen should also be energised with a second transducer containing the same gems and filter. This will improve the biological activity and energy of the blood components, increase the efficiency of the spleen and the cardiovascular system. Also, in some cases, a cooling, calming treatment to the liver with a transducer containing emerald and sapphire at 1.5 Hz is very helpful.

Sciatica. Two transducers containing emerald and sapphire are used with a green filter at 1.5 Hz. The inflamed or irritated section of the spinal nerve on the patients back is targeted (usually around L4 and L5 area). The second transducer is placed at the patient's knee joint of the affected leg. This will give immediate and effective relief of pain and will relax all of the tense muscles and relieve the pressure on the inflamed nerve section. It will also rapidly reduce the inflammation. Transducers containing blue sapphire and a violet filter at 1.5 Hz are likewise just as effective and for some patients are superior. Usually sciatica requires only one treatment of 20 minutes.

Shingles. Herpes zoster is treatable in the first instance by using transducers containing emerald and sapphire with a green filter at 1.5 Hz. Since this condition is mainly confined to older patients, benefit can be gained by stimulating the spleen with a transducer containing diamond and carnelian with an orange filter. This will improve the biological activity of all of the blood components, and increase the efficiency of the spleen and the cardiovascular system. This treatment will also improve the constitution and induce a feeling of well-being.

Sinusitis. A transducer containing diamond and carnelian will be used on the spleen for 20 minutes with an orange filter at 8.3 Hz. This treatment should abate the sinusitis within a few days by aiding the body's immune system to tackle the infection. If this alone is insufficient, the use of a transducer containing sapphire on the nasal and forehead areas with a blue filter at 1.5 Hz should relieve the pain. The use of a transducer containing blue topaz on the throat area for 20 minutes at 8.3 Hz, blue filter is often of great benefit in this disease. If the sinusitis is linked to a throat infection, the practitioner will use a transducer containing sapphire and emerald on the throat area for 20 minutes with a green filter and a frequency of 8.3 Hz.

Stress. The practitioner will check and correct the Assemblage Point location and entry angle and treat this condition as per Anxiety. The practitioner will raise the body's energy by treating the spleen with diamond and carnelian at 8.3 Hz using an orange filter for 20 minutes. This enables the patient to feel more capable to deal with the sources of stress.

Stroke. Paralyses and muscle coordination can be dramatically improved. The practitioner's first consideration is to improve the cardiovascular system and the quality of the blood. To achieve this, the practitioner will use a transducer containing diamond and carnelian with an orange filter at a frequency of 8.5 Hz and will target the patient's spleen. This will effectively stimulate and tone up the patient's blood as it passes through the spleen on the way to liver. The circulation of the blood will carry and distribute the energy to all the organs, glands and extremities of the patient's body including the brain. The patient should be made comfortable sitting or lying on an examination couch. The transducers should be arranged to target the spleen from a distance of approximately 1 to 5 cm. The duration of the treatment should be for approximately 20 minutes once a week and continued for three sessions before proceeding to treat the affected part of the brain. The energised and improved blood will assist in the breaking up of blood clots and in repairing and strengthening any ruptured blood vessels. At the same time as treating the patient's spleen, if the limbs are paralysed, treatment should be given to the groups of muscles which the patient cannot control, using a transducer containing diamond and carnelian with an orange filter at a frequency of 8.5 Hz. For the groups of muscles that are under excess tension, the practitioner will use a transducer containing blue sapphire with a violet filter at a frequency of 8.5 Hz on the second or third output of the apparatus.

After the preliminary treatment of three sessions, treatment can proceed to targeting the brain area that has been affected using a transducer containing diamond and carnelian with an orange filter at a frequency of 8.5 Hz. The second and third lamps used to treat the muscles above should be employed at the same time. This will greatly assist in the regeneration of the nerve pathways to the paralysed limbs or other affected parts such as the vocal cords or facial muscles, etc.

Sun Burns. The practitioner will use transducers containing emeralds and blue sapphires with a green filter at a frequency of 1.5 Hz and with the transducers close to the skin. This is a cooling, calming treatment.

Surgery Trauma. Trauma from surgery, even with the use of modern anaesthetics can be retained at a deep cellular level of consciousness. Unconscious trauma can inflict a change in body posture by causing unsymmetrical muscle tension. Over time this can cause back pain and other skeletal problems. The practitioner will use one or two transducers containing emeralds and blue sapphires with a green filter and a frequency of 1.5 Hz. The practitioner will advise the patient that, as a result of the treatment, he or she will feel relaxed and calm for a day or so. In addition, healing will be accelerated by energising the spleen using a transducer containing diamond and carnelian at a frequency of 8.3 Hz and with an orange filter for 20 minutes. This can aid the healing of the bruising and damage caused by the operation and can increase the patient's general strength to combat the shock to the system.

Tooth Ache. The practitioner will se a transducer containing emeralds and blue sapphires with a green filter and at a frequency of 1.5 Hz. The transducer will be targeted very close to the jaw. If infection is present, yellow sapphire and citrine can be added to the gem mixture in the transducer.

Thrombosis. Clotting of the blood in the limbs is treated using a transducer containing ruby and diamond with a red filter at a frequency of 16.5 Hz. Thrombosis relating to strokes is treated via the spleen and brain with transducers containing diamond and carnelian with an orange filter at a frequency of 8.5 Hz. The treatment to the spleen should always be for the first two or three sessions and confined to the spleen. This will tone up the cardiovascular system and the quality of the blood components. After two or three weekly treatments to the spleen, the affected side or area of the brain can be treated with the same transducers. The same treatment is effective for haemorrhage of the brain and elsewhere in the vascular system.

Urticaria. The practitioner will use transducers containing emerald and sapphire with a green filter at 1.5Hz to calm and cool the liver. The practitioner will also c heck for ingestion of toxic chemicals such as epoxy resins. This condition can be caused by toxic inflammation of the liver. The liver will be several degrees hotter that the spleen. The liver should be cooled and calmed with one or two transducers containing emerald and blue sapphire with a green filter at a frequency of 1.5 Hz. Since it may not be possible to establish the cause of the liver inflammation and since the condition of the blood will be disturbed, it is advisable to give treatment to the spleen with a transducer containing diamond and carnelian fitted with an orange filter. It is expedient to do this at the same time as cooling the liver and the practitioner will therefore use the same frequency of 1.5 Hz. This treatment will often be successful with only a single treatment of 20 minutes, but one ore two follow-up treatments should be given to be conclusive.

Varicose Ulcers. Leg ulcers respond rapidly and patients with ulcers for many years have had them healed with a few treatments. Ulcers are caused by low local cellular biological energy, poor circulation, toxins building up in the leg tissue and other reasons. It is a cardiovascular problem. Ulcers are best treated in the first place with one or more transducers containing diamond and carnelian with an orange filter at 8.5 Hz. This will improve local circulation and raise the biological activity of the effected dermis. The practitioner will use the transducers to target the tissue around the ulcer and generally to stimulate the circulation in the patient's legs and feet. Treatment using transducers containing ruby and diamond at 16.5 Hz is very effective. Therefore, if the healing progress is slower than required this prescription should be used. It is not necessary or desirable to remove the patient's dressings but, if the dressings are removed, the practitioner should use a face mask and disposable gloves and wipe the transducers with an alcohol swab before and after treatment.

A second transducer containing diamond and carnelian with an orange filter at 8.5 Hz should be used to target the patient's spleen. This will effectively stimulate and tone up the patient's blood as it passes through the spleen on the way to the liver. It will also stimulate and improve the functioning of the cardiovascular system. The circulation of the blood will carry and distribute the energy to all the organs, glands and extremities of the patient's body including the brain. This is a stimulating treatment that rapidly increases the patient's biological energy, the effects of this treatment will become apparent within 10 minutes or so by an improvement in the patient's complexion and he or she will report feeling better and more alive.

Viral Infections. The practitioner will use transducers containing diamond and carnelian on the spleen area with a frequency of 8.3 Hz for 20 minutes using an orange filter. The practitioner will also calm the patient's temperature, if necessary, by using a transducer containing sapphire and emerald on the liver using a green filter for 10 minutes at a frequency of 3.3 Hz. If the throat or upper bronchial areas are infected, the practitioner will target those areas using a transducer containing sapphire and emerald at a frequency of 8.3 Hz and green filter for 20 minutes.

Warts. Allopathic medicine considers warts to be a variety of cancer. To treat warts, the practitioner will use transducers containing citrine with a yellow filter at a frequency of 8.5 Hz and will target the wart from about 5 centimetres. This procedure is equally effective for warts on the hands, limbs and possibly the genitals. It is probably effective for warts in the mouth, throat and larynges.

Although the therapy apparatus of the present invention has been designed initially for use on human patients, the apparatus also has veterinary applications. For example, the apparatus may be used for the treatment of dogs and horses. For veterinary purposes, the sizes of the transducers that are employed will depend on the sizes of the animals being treated.

## Claims

1. Therapy apparatus comprising one or more transducers for emitting radiation by means of dielectric resonance when subjected to an electrical stimulus, generating means for providing the stimulus and control means for controlling the generating means whereby radiation emitted from the transducers is controlled so as to synchronise with the radiation generated in a selected diseased or injured area of a body and thereby stimulate or attenuate emissions of radiation by the body in the diseased or injured area.

2. Apparatus as claimed in Claim 1, in which the or each transducer comprises a low voltage medical lamp containing at least one frequency-modulated crystalline mineral substrate.

3. Apparatus as claimed in Claim 2, in which the crystalline mineral substrate comprises one or more selected from the group comprising ruby, carnelian, citrine, emerald, topaz, diamond, sapphire, chrysoberyl, zircon and amethyst.

4. Apparatus as claimed in Claim 3, which includes a transducer containing an emerald substrate having a dielectric resonance frequency in the green spectrum with a wavelength λ of around 565 ηm and/or a blue sapphire substrate having a wavelength λ of around 400 ηm.

5. Apparatus as claimed in Claim 3, which includes a dielectric resonance transducer containing red aluminium chromium oxide (ruby) having a wavelength λ of around 625 ηm, and/or orange silicon dioxide (carnelian with a wavelength λ of around 610 ηm) and/or carbon (diamond with a wavelength λ of around 470 ηm).

6. Apparatus as claimed in Claim 3, which includes a transducer filled with blue sapphires fitted with a dark violet filter and operated at a frequency of 3.3 Hz.

7. Apparatus as claimed in Claim 3, which includes a transducer filled with carnelians and diamonds fitted with an orange filter and operated at a frequency of 8.5 Hz.

8. Apparatus as claimed in Claim 3, which includes a transducer filled with rubies and diamonds fitted with a red filter and operated at a frequency of 16.5 Hz.

9. Apparatus as claimed in any one of the preceding claims, which includes a modulator, a treatment intensity control means for controlling the output of the modulator and a treatment intensity meter for displaying the treatment intensity.

10. Apparatus as claimed in any one of the preceding claims, which includes a tube located adjacent the transducer and means for passing a fluid through the tube.

11. Apparatus as claimed in any one of Claims 1 to 9, which includes a vessel for receiving a medical substrate, means for stirring the medical substrate and means for positioning the transducer so that the medical substrate is subjected to the radiation generated by the transducer.
